# EUROPÄISCHE PATENTSCHRIFT

(11) **EP 2 144 649 B1**
(45) Veröffentlichungstag und Bekanntmachung des Hinweises auf die Patenterteilung: **20.07.2011**
(21) Anmeldenummer: 07816243.5
(22) Anmeldetag: 12.11.2007
(51) Int. Cl.: A61M 5/24

(54) **VERABREICHUNGSVORRICHTUNG MIT FUNKTIONALEM ANTRIEBSGLIED**
ADMINISTERING APPARATUS WITH FUNCTIONAL DRIVE ELEMENT
DISPOSITIF D'ADMINISTRATION À ÉLÉMENT D'ENTRAÎNEMENT FONCTIONNEL

(30) Priorität: 05.04.2007 DE 102007016810
(43) Veröffentlichungstag der Anmeldung: 20.01.2010
(73) Patentinhaber: Tecpharma Licensing AG, 3401 Burgdorf (CH)
(72) Erfinder: TSCHIRREN, Markus, CH-3422 Kirchberg (CH); MOSER, Ulrich, CH-3412 Heimiswil (CH); HIRSCHEL, Jürg, CH-5000 Aarau (CH); BAUMERT, Jan, CH-3455 Grünen (CH); HATTLER, Eric, CH-4500 Solothurn (CH)
(86) Internationale Anmeldenummer: PCT/CH2007/000560
(87) Internationale Veröffentlichungsnummer: WO 2008/122132

(56) Entgegenhaltungen:
- EP-A- 1 066 847
- DE-A1-102004 055 298
- US-B1- 6 793 646

## Beschreibung

Die vorliegende Erfindung betrifft eine Vorrichtung zur Verabreichung eines fluiden Produkts sowie ein Verfahren zur Vorbereitung der Vorrichtung. Insbesondere betrifft die Erfindung eine Vorrichtung zur Verabreichung eines Wirkstoffes aus einem Zweikammerreservoir, das eine Steuereinrichtung zum Steuern des Ablaufes zur Vorbereitung und Verabreichung des fluiden Produkts aufweist.

Aus dem Stand der Technik sind eine Vielzahl von Verabreichungsvorrichtungen bekannt, mit welchen ein flüssiges oder fluides Medikament oder ein anderes fluides Produkt aus einem Behälter, wie z.B. einer Glaskarpule, auf einfache Weise verabreicht werden können. Der Produktbehälter wird in die Verabreichungsvorrichtung eingesetzt, wobei sich ein Antriebsglied, wie etwa eine Kolbenstange, zur Verabreichung des Produkts an einen Stopfen in dem Behälter anschliesst. Durch eine Bewegung des Antriebsglieds relativ zum Behälter der Verabreichungsvorrichtung wird der Stopfen innerhalb des Behälters vorgeschoben, so dass das Medikament durch eine mit dem Behälter verbundene Injektionsnadel ausgeschüttet werden kann. Die Anwendung solcher Verabreichungsgeräte wird dadurch erleichtert, dass die Vorrichtungen für den einmaligen Gebrauch vorgesehen werden, d.h. nachdem ein Behälter geleert wurde, wird die Verabreichungsvorrichtung mitsamt dem Behälter entsorgt. Ein Wechsel des Behälters ist nicht nötig. Weiter ist es zur einfachen Anwendung üblich, dass die Länge des Vorschubs zur Ausschüttung des Medikaments durch bauliche Massnahmen vorgegeben ist und nicht eigens vom Anwender eingestellt werden muss. Zudem sind auch Vorrichtungen bekannt, bei welchen ein gesonderter Entlüflungsschritt vorgesehen ist, durch den eine möglicherweise in dem Behälter befindliche Luftmenge vor der Ausschüttung des Wirkstoffes aus dem Behälter entfernt werden kann. Letztlich gibt es Verabreichungsvorrichtungen, welche auf die Verabreichung von fluiden Produkten aus Zweikammerkarpulen spezialisiert sind. Bei solchen Zweikammerkarpulen ist es notwendig, das zu verabreichende fluide Produkt kurz vor der Anwendung abzumischen, da der Wirkstoff z.B. in fester Form in einer ersten Kammer und ein entsprechendes Lösungsmittel für den Wirkstoff in einer zweiten Kammer vorgesehen ist. Zum Abmischen des fluiden Produkts wird durch Vorschub der Stopfen innerhalb der Karpule eine Fluidverbindung zwischen den beiden Kammern hergestellt, so dass das Lösungsmittel in die Kammer des festen Wirkstoffs gelangen kann. Die Verwendung einer solchen Zweikammerkarpule und deren Abmischen ist z.B. aus EP 0298067 B1 bekannt.

Aus der EP 0911046 B1 ist eine vorgefüllte Spritze bekannt, die zum Abmischen eines Produkts in einem Zweikammerbehälter eine Kolbenstange mit einem Stoppmittel umfasst, durch welches der Ablauf beim Abmischen des Produkts gesteuert wird. Zu diesem Zweck ragt das Stoppmittel seitlich von der Kolbenstange ab, so dass es beim Einschieben der Kolbenstange am Behälterrand anschlägt und einen Stopp der Kolbenstangenbewegung erwirkt. Zu diesem Zeitpunkt ist das Lösungsmittel über einen Bypass in die Kammer des Wirkstoffes gelangt, so dass eine Abmischung des Produkts erfolgt. Durch weiteres Einschieben der Kolbenstange klappt das Stoppmittel in Richtung der Achse der Kolbenstange und fügt sich in eine Aussparung innerhalb der Kolbenstange ein, so dass die Kolbenstange weiter in das Behältnis eingeschoben werden kann. Durch das fortgeführte Einschieben der Kolbenstange kann das Produkt aus dem Behälter ausgeschüttet werden.

Eine ähnliche Vorrichtung ist aus EP 0793973 A2 bekannt. In dieser Ausführuhgsform einer Doppelkammerspritze weist die Kolbenstange an ihrem Umfang einen oder mehrere Vorsprünge auf, welche den Vorschub der Kolbenstange in den Spritzenbehälter hinein kurzzeitig unterbrechen. Die Unterbrechung dient als Anzeige dafür, dass das Lösungsmittel über den Bypass in die Wirkstoffkammer gelangt ist und der Abmischvorgang abgeschlossen ist. Durch weiteres Einschieben der Kolbenstange wird der Widerstand durch die Vorsprünge überwunden und das Produkt kann aus der Spritze ausgeschüttet werden.

Aus der US 6,793,646 B1 ist eine 2-Kammerkarpulen Vorrichtung bekannt, bei der eine automatische Rekonstitution eines Medikaments durchgeführt wird. Eine Hülse, welche eine vorgespannte Feder aufgenommen hat, wird von einem Vorsprung des Gehäuses in einer Ausgangsposition gehalten. Wird das Gehäuse relativ zum der 2-Kammerkarpulen bzw. der Aufnahme der 2-Kammerakarpulen entlang einer Längsachse bewegt, kommt der Vorsprung ausser Eingriff mit der Hülse. Die Hülse kann sich aufgrund der Beaufschlagung der Feder vorwärts bewegen und die Rekonstitution des Medikaments wird bewerkstelligt.

Es ist eine Aufgabe der vorliegenden Erfindung, eine Vorrichtung zur Verabreichung eines fluiden Produkts bereitzustellen, die den Ablauf zur Vorbereitung der Vorrichtung und den Verabreichungsvorgang vereinfacht und koordiniert, einen fehlerhaften Gebrauch der Vorrichtung ausschliesst, und die nur wenige Bauteile umfasst und kostengünstig in der Herstellung ist.

Weiter ist es eine Aufgabe der Erfindung, die Verabreichung eines fluiden Produkts aus einem Zweikammerreservoir zu vereinfachen und sicher zu gestalten.

Eine weitere Aufgabe der Erfindung ist es, ein Verfahren zur Vorbereitung einer Verabreichungsvorrichtung vorzusehen, das die Handhabung der Vorrichtung vereinfacht und eine fehlerfreie Anwendung gewährleistet.

Die Aufgaben werden gelöst durch eine Vorrichtung nach Patentanspruch 1 und durch Verfahren nach Patentansprüchen 17 und 19 erfüllt. Vorteilhafte Ausgestaltungen der Vorrichtung und der Verfahren gehen aus den Unteransprüchen hervor.

Eine Vorrichtung zur Verabreichung eines fluiden Produkts gemäss der Erfindung weist ein Gehäuse auf, in das ein Aufnahmeglied für das fluide Produkt, bzw. für ein Reservoir des fluiden Produkts, einführbar ist. In dem Gehäuse ist ein Antriebsglied gelagert, das relativ zum Aufnahmeglied zur Ausschüttung des fluiden Produkts beweglich ist. Das Gehäuse kann höhlenförmig ausgebildet sein und aus einem oder mehreren Elementen gebildet werden. Das Aufnahmeglied für das fluide Produkt ist beispielsweise als Halter für ein Produktreservoir, wie z.B. eine Zweikammerkarpule, ausgebildet. Vorzugsweise umschliesst das Aufnahmeglied das Produktreservoir und bietet in seinem distalen Bereich eine Aufnahme zum Aufsetzen einer Injektionsnadel, welche eine Fluidverbindung zum Inneren des Produktsreservoirs herstellt. An seinem proximalen Ende wird das Aufnahmeglied an das Gehäuse angeschlossen oder in dieses eingesetzt. Das Aufnahmeglied ist relativ zum Gehäuse entlang einer Längsachse des Gehäuses von einer Anfangsposition in eine eingeführte Position bzw. eine Einfuhrposition, beweglich. Dabei kann das Aufnahmeglied vollständig oder auch nur teilweise in das Innere des Gehäuses eingeführt werden. Zum Einführen des Aufnahmeglieds in das Gehäuse sind Führungseinrichtungen vorgesehen, welche die Bewegung des Aufnahmeglieds gegenüber dem Gehäuse leiten. Hierfür können z.B. Nuten oder Rillen am Gehäuse oder dem Aufnahmeglied und entsprechende Vorsprünge oder Nocken, welche innerhalb der Nuten laufen, an dem anderen Element vorgesehen werden. Vorzugsweise wird die Führungseinrichtung durch eine Gewindeführung vorgesehen. Hierfür ist an der Innenfläche des Gehäuses ein Innengewinde und an der Aussenseite des Aufnahmeglieds ein Aussengewinde vorgesehen, welches in dem Innengewinde des Gehäuses geführt wird.

Demnach wird die Einführbewegung zum Einführen des Aufnahmegliedes in das Gehäuse entlang der Längsachse des Gehäuses in die proximale Richtung durch eine Schraubenbewegung zwischen Gehäuse und Aufnahmeglied ausgeführt. Über die Steigung des Gewindes, kann die Geschwindigkeit der translatorischen Bewegung der Teile zueinander eingestellt werden.

Das in dem Gehäuse gelagerte Antriebsglied dient der Ausschüttung des fluiden Produkts aus dem Produktreservoir, d.h. dem Vorschub eines Stopfens innerhalb des Reservoirs in die distale Richtung. Hierzu ist das Antriebsglied relativ zum Gehäuse in die distale Richtung beweglich. Das Antriebsglied wird beispielsweise durch eine Kolbenstange gebildet. Befindet sich die Verabreichungsvorrichtung in einer Position, in welcher sie zur Verabreichung eines Produkts bereit ist, ragt die Kolbenstange am proximalen Ende aus dem Gehäuse hervor, so dass durch Einschieben der Kolbenstange in die proximale Richtung des Gehäuses eine Vorschubbewegung für den Stopfen innerhalb des Reservoirs erzeugt werden kann. Das Einschieben der Kolbenstange kann dabei manuell erfolgen. Es ist aber auch möglich, eine automatische Ausschüttung vorzusehen, z.B. durch ein Kraftelement wie einer Feder, die zum Vorschub Kraft auf das Antriebsglied ausübt.

Erfindungsgemäss weist das Antriebsglied eine Steuereinrichtung auf, mit welcher der Ablauf bei der Handhabung der Verabreichungsvorrichtung gesteuert werden kann, nämlich die sequenzielle Abfolge von Abmischen, Entlüften, Aufdosieren, Injizieren und Blockieren der Vorrichtung. Die Steuereinrichtung wird durch ein funktionales Antriebsglied gebildet, welches die Abfolge der einzelnen Schritte mechanisch steuert. Hierfür besitzt das Antriebsglied wenigstens ein Halteelement. Das Halteelement hält in der Anfangsposition zwischen Aufnahmeglied und Gehäuse das Antriebsglied relativ zum Gehäuse. In der Halteposition ist daher das Antriebsglied relativ zum Gehäuse nicht in eine proximale Richtung entlang der Längsachse des Gehäuses beweglich. Das Antriebsglied ist in der Halteposition auch derart im Gehäuse gelagert, dass auch eine Bewegung relativ zum Gehäuse in distale Richtung verhindert wird. Hierfür kann beispielsweise ein Anschlag am Antriebsglied an einen Gegenanschlag am Gehäuse oder einem gehäusefesten Teil vorgesehen sein, welche eine Bewegung des Antriebsglieds im Gehäuse in distale Richtung verhindern. Daher ist vorzugsweise das Antriebsglied in der Halteposition sowohl gegen eine Bewegung in distale Richtung als auch in proximale Richtung gegenüber dem Gehäuse gehindert. Es besteht also eine Wirkverbindung derart, dass das Halteelement relativ zum Gehäuse feststeht, d.h. unbeweglich ist.

Gemäss der Erfindung wird durch Einführen des Aufnahmeglieds das Antriebsglied relativ zum Gehäuse gelöst. Die Relativbewegung zwischen Aufnahmeglied und Gehäuse erwirkt somit ein Lösen der Wirkverbindung zwischen Antriebsglied und dem Gehäuse, bzw. einem gehäusefesten Element der Vorrichtung. In dieser gelösten Position, bzw. Löseposition, ist das Antriebsglied relativ zum Gehäuse in die proximale Richtung beweglich. Es ist daher möglich, das Antriebsglied relativ zum Gehäuse in eine Stellung zu bringen, die als aufdosierte Stellung bezeichnet werden kann, aus der ein Ausschütthub relativ zum Gehäuse in distale Richtung ausgeführt werden kann.

Das Halteelement wird zum Halten des Antriebsglieds mit dem Gehäuse gekoppelt. Dies kann durch unmittelbaren Kontakt zwischen Halteelement und Gehäuse erfolgen, aber auch durch gehäusefeste Zwischenelemente innerhalb der Verabreichungsvorrichtung vermittelt werden. Zum Lösen des Antriebsgliedes aus der Halteposition wird das Antriebsglied vom Gehäuse oder einem gehäusefesten Teil entkoppelt. Die Halteposition wird z.B. dadurch realisiert, dass das Halteelement gegen einen gehäusefesten Axialanschlag stösst, der eine axiale Bewegung entlang der Längsachse des Gehäuses, des Halteelements und somit des Antriebselements verhindert.

In einer bevorzugten Ausführungsform ist das Halteelement des Antriebsglieds als wenigstens ein radial vom Antriebsglieds abragender flexibler Fortsatz ausgebildet. Der flexible Fortsatz kann z.B. als Arm oder Spange ausgebildet sein, die integraler Bestandteil des Halteelements sein können oder angeformt sein können. Demnach ragt das Haltelement radial von dem Antriebsglied ab, ist jedoch durch seine Flexibilität relativ zum Antriebsglied beweglich. Das Halteelement weist jedoch eine ausreichende Steifigkeit auf, so dass eine Spannkraft erforderlich ist, um es aus seiner Grundstellung relativ zum Antriebsglied auszulenken. Der flexible Fortsatz ist daher federelastisch an dem Halteelement angeordnet. Durch Aufbringen einer Kraft kann das Halteelement, bzw. der Fortsatz, gegenüber dem Antriebsglied bewegt werden, vorzugsweise radial in Richtung zur Achse des Antriebsglieds. In einer bevorzugten Ausführungsform ragt der flexible Fortsatz im Wesentlichen in Richtung der Einführbewegung des Aufnahmeglieds von dem Halteelement ab. Dies kann z.B. durch ein gabelförmig abgespreiztes Ärmchen vorgesehen werden, das an seinem distalen Ende am Antriebsglied fest ist und mit seinem proximalen Ende von dem Antriebsglied abragt. In der Halteposition stösst das proximale Ende des Ärmchens an einen gehäusefesten Anschlag oder eine Kante und verhindert somit eine Bewegung des Antriebsgliedes in proximale Richtung.

Zum Lösen der Halteposition, bzw. des Halteeingriffs oder der Wirkverbindung, beim Einführen des Aufnahmeglieds greift das Aufnahmeglied an dem Halteelement an und bewegt es in die Löseposition. Ist das Halteelement als flexibler Forstsatz in Form eines vom Antriebsglied abgespreizten Ärmchens ausgebildet, kann sich das Aufnahmeglied bei der Einführbewegung in das Gehäuse z.B. über das Ärmchen schieben und somit eine radial nach innen wirkende Kraft auf das Ärmchen ausüben. Das Halteelement wird somit aus seiner Halteposition bewegt und damit das Antriebsglied für eine Bewegung in proximale Richtung freigegeben.

In einer Ausführungsform weist die Vorrichtung zur Verabreichung eines fluiden Produkts ein Produktreservoir in Form eines Zweikammerreservoirs auf. Das Zweikammerreservoir umfasst, wie aus dem Stand der Technik bekannt, eine erste Kammer für einen Wirkstoff, die durch einen ersten Stopfen begrenzt ist, und eine zweite Kammer für ein Lösungsmittel, die durch den ersten und einen zweiten Stopfen begrenzt ist. Die Kammern sind via einem Bypass in der Wand des Reservoirs durch Verschiebung der Stopfen verbindbar, um ein Abmischen des Wirkstoffs und des Lösungsmittels zu ermöglichen. Gemäss der Erfindung ist das Halteelement in der Halteposition derart ausgebildet, dass das Antriebsglied beim Einführen des Aufnahmeglieds gegen den zweiten Stopfen stösst und diesen relativ zum Aufnahmeglied beim Einführen verschiebt bis das Aufnahmeglied in einer Abmischposition ist, in der das fluide Produkt mit dem Lösungsmittel vermischt werden kann. Anschliessend an das Abmischen wird vorzugsweise eine Entlüftung des Produktreservoirs durchgeführt, um in dem Reservoir verbliebene Luft vor einer Verabreichung des Produkts aus dem Reservoir zu eliminieren. Ein solcher Entlüftungsvorgang kann natürlich auch bei Einkammerreservoiren notwendig sein und durchgeführt werden. Hierfür wird das Aufnahmeglied von der Abmischposition, oder der Anfangsposition im Falle einer Einkammerposition, in eine Entlüftungsposition weiter in proximaler Richtung in das Gehäuse eingeführt. Dabei wird der Stopfen innerhalb des Reservoirs weiter in distale Richtung verschoben. Zur Entlüftung ist es notwendig, eine Nadeleinheit auf das Aufnahmeglied aufzusetzen, die eine Verbindung zwischen dem Reservoir und der Umgebung herstellt, so dass die Luft beim Verschieben des Stopfens über die Nadel aus dem Reservoir entweichen kann.

Erfindungsgemäss verbleibt demnach das Antriebsglied in der Halteposition gegenüber dem Gehäuse so lange, bis der Abmischvorgang in dem Zweikammerreservoir und vorzugsweise auch der Entlüftungsvorgang abgeschlossen sind. Der Abmisch- und Entlüftungsvorgang erfolgt vorzugsweise durch Eindrehen des Aufnahmeglieds entlang der Gewindeführung innerhalb des Gehäuses. Beim Abmisch- oder Entlüftungsvorgang, d.h. sobald das Aufnahmeglied weit genug in das Gehäuse eingeschraubt ist, wird vorzugsweise gleichzeitig die Halteposition des Antriebsglieds gelöst, indem die Blockierung des Antriebsglieds mittels des Halteelements an dem Gehäuse oder dem gehäusefesten Teil aufgehoben wird. Das Aufnahmeglied befindet sich dann in einer eingeführten Position, in der es vorzugsweise nicht weiter in das Gehäuse eingeschraubt werden muss oder kann. Es ist auch möglich zum Lösen der Halteposition, das Aufnahmeglied über die Abmisch- oder Entlüftungsposition hinaus um eine geringe Strecke weiter in das Gehäuse einzuführen bis die gelöste Position erreicht ist.

In einer Ausführungsform der Erfindung ist das Antriebsglied in der Halteposition derart innerhalb des Gehäuses gelagert, dass das proximale Ende des Antriebsgliedes, das als Antriebsknopf ausgebildet sein kann, innerhalb des Gehäuses zu liegen kommt. Das Antriebsglied ist daher in der Position der Verabreichungsvorrichtung vor dem Einführen des Aufnahmeglieds, bzw. eines Zweikammerreservoirs, und vorzugsweise auch vor einem Entlüftungsvorgang oder Abmischvorgang vor einem Zugriff durch den Anwender geschützt. Eine Betätigung des Verabreichungsgeräts zum Ausschütten des fluiden Produkts ist nicht möglich. Vorzugsweise schliesst der Antriebsknopf am Antriebsglied bündig mit dem proximalen Gehäuseende ab. Das Antriebsglied, bzw. der Antriebsknopf, kann während dem Einführen des Aufnahmegliedes zum Entlüften oder Abmischen des Reservoirs in dieser Ausgangsposition verbleiben. Es kann jedoch bereits während der Einführbewegung für das Entlüften oder Abmischen durch diese Bewegung aus dem Gehäuse ausgefahren werden.

Gemäss einer bevorzugten Ausführungsform der Erfindung besitzt das Antriebsglied ein Aktivierungselement, an dem das Aufnahmeglied bei der Einführbewegung angreift und das Antriebsglied relativ zum Gehäuse in eine aktivierte Position bewegt. Vorzugsweise wird diese aktivierte Position definiert durch das Ausfahren des Antriebsglieds aus dem proximalen Ende des Gehäuses. Es wäre aber auch möglich, dass das Antriebsglied ein Betätigungselement umfasst, das durch seitliche Bewegung in eine aktivierte Position gebracht wird, z.B. zum anschliessenden Auslösen einer Antriebsfeder. Zur Betätigung des Aktivierungselements kann es einen Axialanschlag aufweisen, der bei der Einführbewegung an dem Aufnahmeglied anstösst. Beispielsweise kann das Aktivierungselement durch einen Fortsatz ausgebildet sein, der in distale Richtung von dem Antriebsglied abragt. Wird das Aufnahmeglied in das Gehäuse eingeführt, kann das Aktivierungselement an das proximale Ende des Aufnahmeglieds, bzw. an dessen Kante anschlagen. Durch eine Fortsetzung der Einführbewegung wird die Bewegung über das Aktivierungselement auf das Antriebsglied übertragen und dieses gemeinsam mit dem Aufnahmeglied in proximale Richtung verschoben. Das Aktivierungselement kann flexibel ausgebildet sein, vergleichbar mit dem Halteelement, und eine Vorspannung aufweisen, so dass es entgegen der Vorspannung aus seiner Anschlagposition am Aufnahmeglied herausbewegt werden kann. Beispielsweise kann hierzu das Aktivierungselement radial in Richtung der Achse des Antriebsglieds gebogen werden. Hierfür kann ein Führungsvorsprung z.B. am Gehäuse vorgesehen sein. In dieser eingebogenen Stellung weicht das Aktivierungsglied dem Rand des Aufnahmeglieds nach innen aus, so dass das Antriebsglied relativ zum Aufnahmeglied in die distale Richtung verschoben werden kann.

In dieser aktivierten Position kann also das Antriebsglied relativ zum Gehäuse und zum Aufnahmeglied in die distale Richtung verschoben werden, so dass es einen Ausschütthub ausführen kann. Durch den Ausschütthub wird der Stopfen in dem Produktreservoir verschoben und Produkt aus dem Reservoir durch eine Injektionsnadel ausgeschüttet. In dieser Ausschütt- oder Endposition nach einer Verabreichung des fluiden Produkts liegt das Aktivierungselement innerhalb des Aufnahmeglieds. Vorzugsweise kommt auch der Antriebsknopf des Antriebsglieds, bzw. das Ende des Antriebsglieds, wieder innerhalb des Gehäuses zu liegen.

In der Endposition stösst das Halteelement gegen den gehäusefesten Anschlag. D.h. das Halteelement befindet sich wieder in seiner ausgelenkten Position, wie zu Beginn, bevor das Aufnahmeglied in das Gehäuse eingeführt wurde. Das Halteglied verhindert somit wieder eine proximale Bewegung des Antriebsglieds gegenüber dem Gehäuse. Dabei kommt nun das Halteelement in diese Endposition im Wesentlichen innerhalb des Aufnahmeglieds zu liegen. In der Endposition ist die Verabreichungsvorrichtung gegen jegliche weitere Betätigung blockiert. Dies erfolgt durch den Anschlag zwischen Halteelement und Gehäuse und der Verrastung des Aufnahmeglieds zum Gehäuse nach dem vollständigen Einführen des Aufnahmeglieds in das Gehäuse.

Es ist möglich, an dem Antriebsglied, vorzugsweise am Halteelement des Antriebsgliedes, ein Begrenzungselement vorzusehen, das eine Bewegung des Antriebsglieds in proximale Richtung relativ zum Gehäuse durch Anschlag an einem gehäusefesten Teil begrenzt. Das Begrenzungselement ist z.B. als Absatz am Halteelement ausgebildet, bei der Einführbewegung des Aufnahmeglieds kann das Halteelement aus der Halteposition ausgelenkt werden, so dass mittels des Aktivierungselements das Antriebsglied in proximale Richtung relativ zum Gehäuse herausgeschoben werden kann. Das Herausschieben wird jedoch durch den Absatz begrenzt, der an den gehäusefesten Teil anschlägt, sobald das Antriebsglied um einen Ausführhub aus dem Gehäuse herausgefahren wurde. Vorteilhafterweise dient der Absatz auch als Führungskulisse für das Aufnahmeglied beim Betätigen des Halteelements, d.h. beim Auslenken des Halteelements aus der Halteposition in die gelöste Position.

Wird das Antriebsglied zur Ausschüttung des Produkts relativ zum Gehäuse und zum Aufnahmeglied in distale Richtung verschoben, wird das Halteelement bevorzugt in das Innere des Aufnahmeglieds eingeschoben. Um zu gewährleisten, dass das Halteelement in seine Halteposition zurückkehren kann, ist in dem Aufnahmeglied eine Aussparung vorgesehen, in die der Absatz des Halteelements in der Endposition eingreifen kann. So wird ein sicheres Zurückstellen des Halteelements in die Halteposition gewährleistet und die Verabreichungsvorrichtung vor weiterem Gebrauch blockiert.

Ein Verfahren zur Vorbereitung einer Verabreichungsvorrichtung gemäss der Erfindung umfasst folgende Schritte. Das Aufnahmeglied, bzw. das Reservoir, wird von einer Ausgangsposition bis zu einer Entlüftungsposition in das Gehäuse eingeführt. Dabei legt das Aufnahmeglied eine erste Einfuhrstrecke zurück. Während dem Zurücklegen dieser ersten Einfuhrstrecke wird das Antriebsglied relativ zum Gehäuse in der Halteposition gehalten und ein Stopfen innerhalb des Reservoirs vorgeschoben. Im nächsten Schritt wird durch weiteres Einführen des Aufnahmeglieds in das Gehäuse das Antriebsglied aus der Halteposition gelöst. Das Lösen der Halteposition erfolgt während des Vorschubs des Aufnahmeglieds relativ zum Gehäuse über eine zweite Einfuhrstrecke. Das Antriebsglied befindet sich nun in einer gelösten Position, in der es nicht mehr relativ zum Gehäuse gegen eine Bewegung in proximaler Richtung gehalten wird. In einem weiteren Schritt wird durch nochmals weiteres Einführen des Aufnahmeglieds das Antriebsglied relativ zum Gehäuse in proximaler Richtung ausgefahren. Das Ausfahren des Antriebsglieds erfolgt während der Bewegung des Aufnahmeglieds über eine dritte Einfuhrstrecke. Das Aufnahmeglied befindet sich nach diesem Schritt, d.h. dem Überwinden der dritten Einfuhrstrecke, in einer eingeführten Position und das Antriebsglied befindet sich in einer aktivierten Position.

Es wird bevorzugt, dass das Verfahren zur Vorbereitung der Verabreichungsvorrichtung einen Schritt umfasst, nach dem während dem Einführen des Aufnahmeglieds ein Abmischen innerhalb eines Zweikammerreservoirs erfolgt, indem ein Stopfen durch das Antriebsglied innerhalb des Zweikammerreservoirs vorgeschoben wird. Das Aufnahmeglied wird in diesem Schritt von der Ausgangsstellung in eine Abmischstellung verschoben. Dieser Abmischvorgang kann während der oben beschriebenen ersten Einfuhrstrecke des Aufnahmeglieds erfolgen. An diesen Abmischweg schliesst sich sodann der Vorschub des Aufnahmeglieds von der Abmischposition in die Entlüftungsposition an. Der Entlüftungsvorgang kann ebenfalls während der ersten Einfuhrstrecke des Aufnahmeglieds erfolgen.

Das Verfahren gemäss der vorliegenden Erfindung zur Vorbereitung der Verabreichungsvorrichtung stellt eine Ablaufsteuerung für den Ablauf der einzelnen notwendigen Schritte bei der Bereitstellung der Verabreichungsvorrichtung für einen Anwender dar. Die erfindungsgemässe Ausgestaltung des Antriebsglieds mit einem Halteelement oder auch einem Aktivierungselement dient dabei als Steuereinrichtung für die Steuerung der einzelnen Schritte. Insbesondere bei Wirkstoffen, welche in getrocknetem Zustand gelagert werden müssen, und erst kurz vor der Verabreichung durch Injektion in einem Lösungsmittel gelöst werden, ist es wesentlich, dass die einzelnen Schritte bei der Vorbereitung der Verabreichungsvorrichtung sorgfältig und exakt ausgeführt werden. Mit Hilfe des Verfahrens der Erfindung werden die einzelnen Vorbereitungsschritte des Abmischens, Entlüftens, Aufdosierens, Injizierens und Blockierens der Vorrichtung auf einfache Weise in Reihe geschaltet. Es ist nicht möglich, das ein Anwender einen Schritt vergisst, unsorgfältig ausführt oder überspringt. Durch das Blockieren des Pens nach der Verabreichung wird sichergestellt, dass keine unsachgemässe Handhabung mit einer bereits gebrauchten Verabreichungsvorrichtung ausgeführt wird.

Nach einem weiteren Aspekt der vorliegenden Erfindung ist bei der Vorrichtung zur Verabreichung eines fluiden Produkts eine Rast-, bzw. Anzeigeeinrichtung, für das Einführen des Aufnahmeglieds in das Gehäuse vorgesehen. Um einen Missbrauch des Geräts zu vermeiden, wird zum einen sichergestellt, dass eine einmal begonnene Einfuhr des Aufnahmeglieds in das Gehäuse nach dem Abschluss eines jeden einzelnen Vorbereitungsschrittes nicht rückgängig gemacht werden kann. Zum andern wird der Abschluss der einzelnen Vorbereitungsschritte durch eine Anzeige angezeigt. Dabei kann die Anzeige z.B. durch eine visuelle Markierung oder einen akustisch wahrnehmbaren Ton gegeben sein. Hierfür sind beispielsweise Arretierungsmittel vorgesehen, durch welche das Aufnahmeglied nach jedem Vorbereitungsschritt gegen eine Bewegung entgegen der Einfuhrrichtung blockiert wird. Dabei ist es wahlweise auch möglich, die Arretierung lösbar auszubilden, sollte dies sinnvoll erscheinen. Im Falle einer Gewindeführung zwischen Gehäuse und Aufnahmeglied bildet die Arretierung beispielsweise eine Rückdrehsicherung gegen ein Wiederherausdrehen des Aufnahmegliedes aus dem Gehäuse. Eine solche Rückdrehsicherung kann beispielsweise durch einen Schnapparm am Aufnahmeglied oder am Gehäuse realisiert werden, der in der Rückdrehrichtung in eine Ausnehmung in dem Gehäuse bzw. dem Aufnahmeglied einrastet, in der Vorwärtsbewegung jedoch aus der Ausnehmung gleiten kann. Eine solche Rückdrehsicherung ist z.B. bei Beendigung des Abmischvorgangs und des Entlüftungsvorgangs vorgesehen. Zudem kann eine solche Rückdrehsicherung bereits beim Einsetzen des Aufnahmeglieds in das Gehäuse vorgesehen werden. D.h. auch in der Ausgangsposition ist das Aufnahmeglied bereits gegen ein vollständiges Herausdrehen aus dem Gehäuse durch eine Arretierung gesichert. Die Verrastung bildet gleichzeitig eine Anzeige, dass der entsprechende Vorbereitungsschritt abgeschlossen ist. In der eingeführten Position nach dem Abmischen und Entlüften kann die Arretierung derart ausgebildet sein, dass auch eine Bewegung in proximaler Richtung, d.h. eine Einschraubbewegung, blockiert wird. Demnach ist das Aufnahmeglied in der Einfuhrposition sowohl gegen ein Ein- wie auch ein Ausführen, bzw. Ein- oder Ausdrehen, gesichert. Alternativ kann das Aufnahmeglied durch eine Verrastung z.B. mit dem Begrenzungselement des Antriebsglieds vorgesehen sein, wie vorher beschrieben.

Vorteilhafterweise werden die Arretierungen, bzw. Rückdrehsicherungen derart ausgebildet, dass beim Einrasten oder Einschnappen in die Arretierung zugleich ein akustisches Signal entsteht, so dass der Abschluss der einzelnen Vorbereitungsschritte hörbar wird. Einem Anwender wird daher die Abfolge der einzelnen Vorbereitungsschritte akustisch angezeigt.

Die vorliegende Erfindung wird anhand der Zeichnung genauer erläutert. Aus der Zeichnung offenbar werdende Merkmale zur Ausgestaltung und Funktion der erfindungsgemässen Verabreichungsvorrichtung gehören zum Umfang der Erfindung und dienen als Offenbarung der Merkmale der Erfindung. In der Zeichnung stellen dar:
- Figur 1A:: Verabreichungsvorrichtung gemäss der Erfindung in einer Ausgangsposition,
- Figur 1B:: Detailansicht der Verabreichungsvorrichtung aus Figur 1A in einer um 90° gedrehten Ansicht,
- Figur 2:: Verabreichungsvorrichtung nach der Erfindung in einer Abmischposition,
- Figur 3:: Verabreichungsvorrichtung nach der Erfindung in einer Entlüflungsposition,
- Figur 4A:: Verabreichungsvorrichtung nach der Erfindung in einer eingeführten Position,
- Figur 4B:: Detailansicht analog Figur 1B in der eingeführten Position,
- Figur 5A:: Verabreichungsvorrichtung nach der Erfindung in einer Endposition,
- Figur 5B:: Detailansicht analog Figur 1B in der Endposition,
- Figur 6A:: Längsschnitt durch die Verabreichungsvorrichtung mit Rasteinrichtung und
- Figur 6B:: Verabreichungsvorrichtung nach Figur 6A in einer Entlüftungsposition,

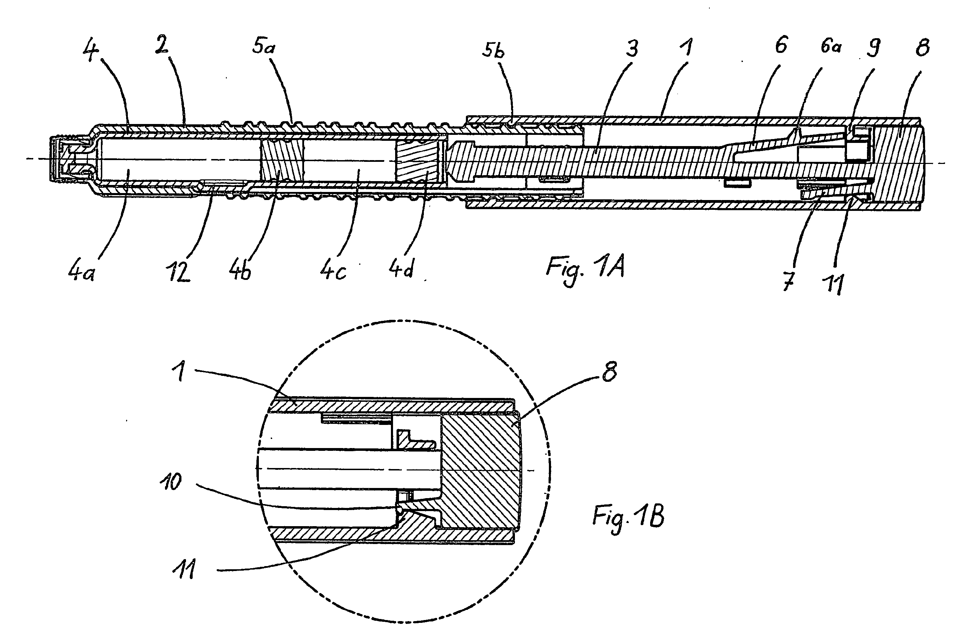

In Figur 1A ist eine Vorrichtung zur Verabreichung eines fluiden Produkts gemäss der vorliegenden Erfindung in einer Ausgangsposition gezeigt. Die Vorrichtung weist ein Gehäuse 1, ein Aufnahmeglied 2 und ein Antriebsglied 3 auf. Die Vorrichtung gemäss der Erfindung kommt allein mit diesen drei Bauteilen aus. Lediglich ein Produktreservoir mit einem gewünschten zu verabreichenden Produkt und eine passende Injektionsnadel sind noch zu ergänzen. Es ist ein Vorteil der Erfindung, dass die Verabreichungsvorrichtung mit nur wenigen einzelnen Bauteilen auskommt. Dadurch werden die Kosten zur Fertigung und zum Zusammenfügen der Bauteile reduziert.

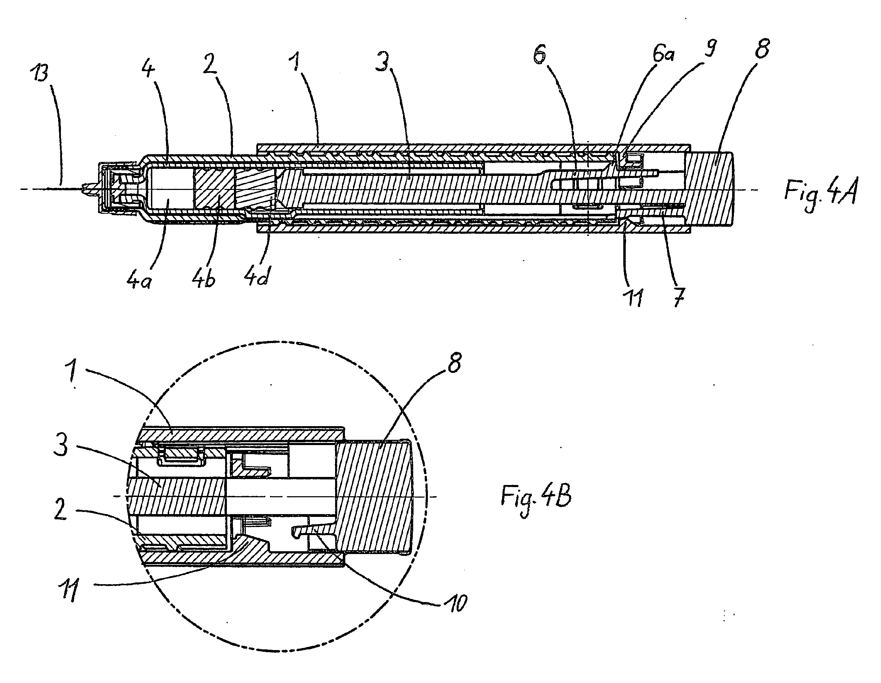

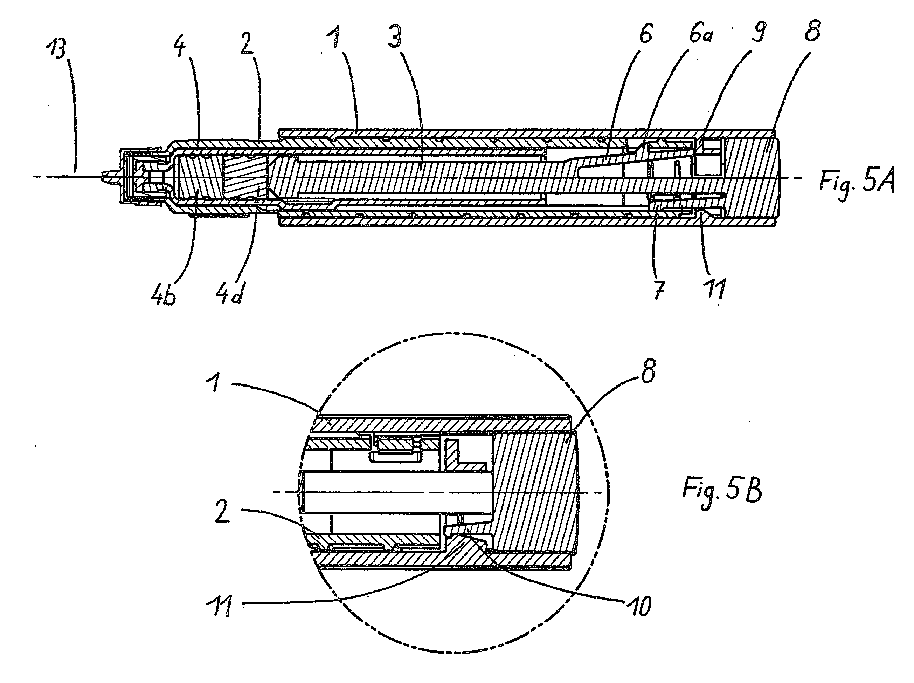

Gemäss dem dargestellten Ausführungsbeispiel ist das Produktreservoir ein Zweikammerreservoir, das eine erste Kammer 4a, die durch einen Stopfen 4b begrenzt ist, und eine zweite Kammer 4c umfasst, die durch den ersten Stopfen 4b und einen Stopfen 4d begrenzt ist. Im Umfang der Wand des Produktreservoirs ist zudem ein Bypass 12 (vgl. Figuren 4a und 5a) vorgesehen, durch den wie aus dem Stand der Technik bekannt, Lösungsmittel aus der zweiten Kammer 4c in die Produktkammer 4a umgeleitet werden kann.

Das Aufnahmeglied 2 ist an seinem proximalen Ende teilweise in das distale Ende des Gehäuses 1 eingesetzt. Zwischen dem Gehäuse 1 und dem Aufnahmeglied 2 ist eine Führungsstruktur vorgesehen, welche eine Relativbewegung zwischen Gehäuse 1 und Aufnahmeglied 2 führt. Die Führungsstruktur umfasst eine gewindeartig auf der Aussenumfangsfläche des hülsenförmigen Aufnahmeglieds 2 umlaufende Führungsnut 5a. Auf die Innenumfangsfläche des ebenfalls hülsenförmig ausgebildeten Gehäuses 1 ist ein Führungsnocken 5b vorgesehen, der innerhalb der Führungsnut 5a geführt ist. Der Führungsnocken 5b kann auch als Führungsschiene oder als Führungssegment ausgebildet sein. Die Führungsstruktur 5a, 5b ist in diesem Ausführungsbeispiel als Gewindeführung ausgeführt. Durch eine Rotationsbewegung des Aufnahmeglieds 2 relativ zum Gehäuse 1 wird das Aufnahmeglied 2 in proximaler Richtung axial in das Gehäuse 1 eingeführt.

Das Antriebsglied 3 ist im Wesentlichen stangenförmig ausgebildet. In dem Ausgangszustand gemäss Figur 1A kommt das distale Ende des Antriebsglieds 3 gegenüber dem proximalen Stopfen 4d des Produktreservoirs 4 zu liegen.

Das Verabreichungsgerät in der Ausgangsposition gemäss Figur 1A entspricht einem Auslieferungszustand, in dem sich die Vorrichtung befindet, wenn sie an einen Anwender ausgegeben wird.

Das Antriebsglied 3 besitzt an seinem proximalen Ende ein Halteelement 6 und ein Aktivierungselement 7. Das Halteelement 6 und das Aktivierungselement 7 sind vorzugsweise integraler Bestandteil des Antriebsglieds 3. Es ist jedoch auch möglich, das Halteelement 6 und das Aktivierungselement 7 an den stangenförmigen Bereich des Antriebsglieds anzufügen. Weiter weist das Antriebsglied 3 am proximalen Ende eine Verdickung auf, welche als Antriebsknopf 8 bezeichnet werden kann. Der Antriebsknopf 8 ist in der Ausgangsposition für einen Anwender unzugänglich, da er im Gehäuse 1 versenkt ist.

Das Halteelement 6 und das Aktivierungselement 7 ragen seitlich als flexible Fortsätze oder Arme vom Antriebsglied ab. Grundsätzlich ist es möglich auch zwei oder mehr solcher Arme am Antriebsglied vorzusehen. Das Halteelement 6 ragt in proximaler Richtung und das Aktivierungselement in distaler Richtung vom Antriebsglied 3 ab.

In der Ausgangsposition gemäss Figur 1A kommt das Halteelement 6 gegenüber einem nach innen ragenden Vorsprung, bzw. einer Kante, 9 des Gehäuses in axialer Richtung zu liegen. Das proximale Ende des Halteelements stösst demnach in axialer Richtung gegen den Gehäusevorsprung 9. In dieser Position ist das Halteelement in seiner Grundstellung. Das Halteelement ist jedoch derart flexibel vorspannbar, dass es in radialer Richtung zur Achse des Antriebsglieds 3 hin bewegt werden kann. Das Aktivierungsglied 7 ragt schräg in distale Richtung von dem Antriebsglied 3 ab. Das Aktivierungselement ist in der Ausgangsposition in einem entspannten Zustand, kann jedoch flexibel in radialer Richtung zur Antriebsgliedachse ausgelenkt werden. Sie übernehmen bei der Vorbereitung der Verabreichungsvorrichtung unterschiedliche Funktionen zur Ablaufsteuerung der einzelnen Vorbereitungsschritte. Sie bilden gemeinsam mit dem Antriebsglied die Steuereinrichtung für die Ablaufsteuerung.

Figur 1B zeigt eine Detailansicht des proximalen Bereichs der Verabreichungsvorrichtung gemäss Figur 1A, in der die Vorrichtung um 90° gedreht ist. Es ist ein Sicherungselement 10 erkennbar, welches mit einem Vorsprung 11 an dem Gehäuse 1 zusammenwirkt. Das Sicherungselement 10 und der Vorsprung 11 bilden zusammen eine Klemmverbindung, um das Antriebsglied 3 zusätzlich zu der Blockierung durch den Anschlag zwischen Halteelement 6 und Gehäusevorsprung 9 sowie dem Anschlag zwischen Antriebsknopf 8 und Gehäusevorsprung 9 zu sichern.

Der Anschlag zwischen dem Halteelement 6 und dem Gehäusevorsprung 9 in axialer Richtung bedingt eine Blockierung der Bewegung des Antriebsglieds in proximaler Richtung relativ zum Gehäuse. Der Anschlag zwischen dem Antriebsknopf 8 und dem Gehäusevorsprung 9 bewirkt hingegen eine Blockierung des Antriebsglieds 3 in distaler Richtung relativ zum Gehäuse.

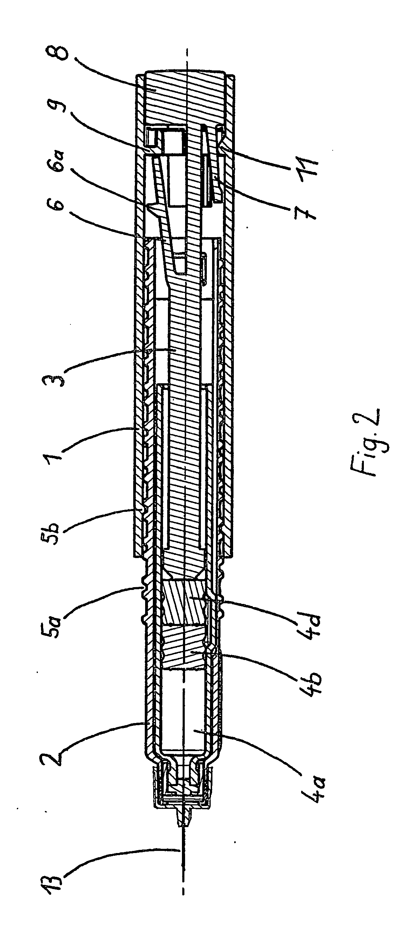

In Figur 2 ist die erfindungsgemässe Verabreichungsvorrichtung in einer Abmischposition gezeigt. Auf das distale Ende der Vorrichtung ist eine Injektionsnadel 13 aufgesetzt. Aus der Ausgangsposition gemäss Figur 1A wird das Aufnahmeglied 2 relativ zum Gehäuse 1 in proximaler Richtung ins Innere des Gehäuses eingeführt bzw. entlang der Führungsstruktur 5a, 5b eingeschraubt. Dabei bleibt das Antriebsglied 3 relativ zum Gehäuse durch die Blockierung zwischen Halteglied 6 und Gehäusevorsprung 9 und auch durch die Sicherung zwischen dem Sicherungselement 10 und dem Vorsprung 11 relativ zum Gehäuse fixiert. Durch das Einführen des Aufnahmeglieds 3 trifft das distale Ende des Antriebsglieds 3 auf den Stopfen 4d des Produktreservoirs 4 und schiebt diesen bei Fortsetzung der Einführbewegung innerhalb des Produktreservoirs in distale Richtung. Dadurch wird zunächst die Antriebskraft durch das Lösungsmittel in der Kammer 4c auf den Stopfen 4b übertragen, so dass beiden Stopfen 4b und 4d distal angetrieben werden. Sobald der Stopfen 4b im Bereich des Bypasses zu liegen kommt, bleibt der Stopfen 4b relativ zum Reservoir 4 in Ruhe. Der Stopfen 4d hingegen wird weiter angetrieben, so dass das Lösungsmittel aus der Kammer 4c über den Bypass in die Kammer 4a gelangt und einen dort befindlichen Wirkstoff lösen kann. Der Stopfen 4d wird solange angetrieben, bis er auf den Stopfen 4b trifft. Dadurch wird die Abmischposition des Verabreichungsgeräts erreicht. Während diesem Vorgang wird das Aufnahmeglied 2 fortwährend weiter in das Gehäuse 1 eingeführt. Das Antriebsglied 3 bleibt jedoch in Ruhe.

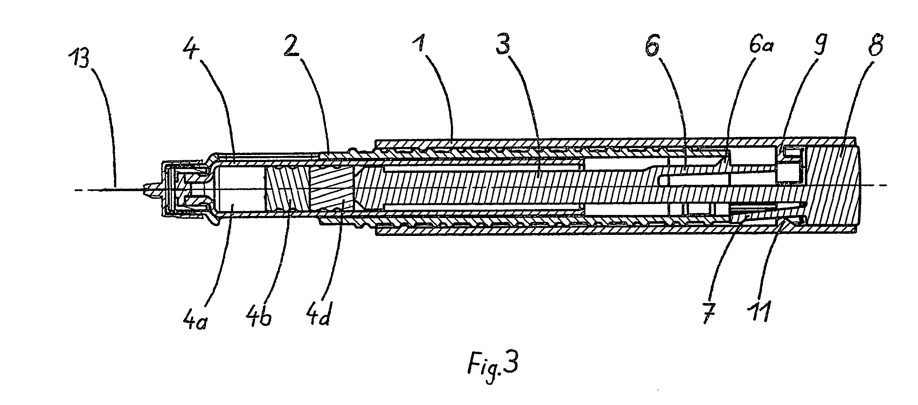

In Figur 3 ist die erfindungsgemässe Verabreichungsvorrichtung in einer Entlüftungsposition gezeigt. Aus der Abmischposition nach Figur 2 wird das Aufnahmeglied 2 weiter in proximaler Richtung in das Gehäuse 1 eingeführt. Das Aufnahmeglied 2 wurde bis hier über eine erste Einfuhrstrecke in das Gehäuse eingeführt, wobei das Halteelement 2 in der Halteposition verbleibt Der proximale Rand des Aufnahmeglieds 2 stösst nun gegen einen Absatz 6a am Halteglied 6. Der Absatz 6a weist eine schräge Ebene auf, die gegenüber dem proximalen Ende des Aufnahmeglieds ausgerichtet ist. Wird das Aufnahmeglied 2 weiter über eine zweite Einfuhrstrecke in das Gehäuse eingeführt, stösst das proximale Ende gegen die schräge Fläche des Absatzes 6a und gleitet an dieser Fläche ab. Dabei wird das Halteelement 6 in radialer Richtung zur Achse des Antriebsglieds 3 hin ausgelenkt. Das Halteelement 6 in Form eines Funktionsarmes wird aus dem Anschlag mit dem Gehäusevorsprung 9 ausgelenkt. Von der Abmischposition in diese gelöste Position, in der das Halteelement 6 des Antriebsglieds 3 aus seiner Halteposition zum Halten des Antriebsglieds 3 relativ zum Gehäuse 1 heraus ausgelenkt wird, legt das Aufnahmeglied 2 eine zweite Einfuhrstrecke zurück, die auch als Freigabestrecke bezeichnet werden kann. Sobald das Halteelement 6 aus dem Anschlag mit dem Gehäusevorsprung 9 ausgelenkt wird, trifft das proximale Ende des Aufnahmeglieds 2 gegen das distale Ende des Aktivierungselements 7. Das Antriebsglied 3 kann nun in proximaler Richtung gegenüber dem Gehäuse 1 über eine dritte Einfuhrstrecke bewegt werden. Während einem weiteren Vorschub des Aufnahmeglieds 2 gegenüber dem Gehäuse 1 zur Auslenkung des Halteelements 6 werden gleichzeitig auch die Stopfen 4b und 4d weiter in distale Richtung des Produktreservoirs 4 verschoben. Durch die Injektionsnadel 13 kann daher überflüssige Luft aus der Produktkammer 4a entweichen. Grundsätzlich wäre es auch denkbar, den Entlüftungsvorgang beim Zurücklegen der ersten Einfuhrstrecke abzuschliessen und nur das Lösen der Halteposition des Halteelements während der zweiten und dritten Einfuhrstrecke durchzuführen.

In Figur 4A ist die erfindungsgemässe Verabreichungsvorrichtung in einer eingeführten Position gezeigt, in der das Aufnahmeglied nach Zurücklegen der dritten Einfuhrstrecke vollständig, d.h. soweit wie erforderlich, in das Gehäuse in proximaler Richtung eingeführt ist. Beim Überwinden dieser dritten Einfuhrstrecke wird das Antriebsglied 3 nicht mehr von dem Halteelement 6 gegenüber dem Gehäuse gehalten. Durch den Anschlag zwischen dem proximalen Ende des Aufnahmeglieds 2 und dem distalen Ende des Aktivierungselements 7 wird das Antriebsglied 3 über die dritte Einfuhrstrecke relativ zum Gehäuse in proximaler Richtung mitbewegt. Dabei wird der Antriebsknopf 8 aus dem proximalen Ende des Gehäuses 1 herausgefahren und ist nun für einen Benutzer zugänglich. Bei der Relativbewegung des Antriebsgliedes 3 und damit des Aktivierungselements 7 gegenüber dem Gehäuse 1 gleitet das Aktivierungselement 7 entlang der nach innen ragenden Kante des Vorsprungs 11 und wird dadurch nach innen ausgelenkt. Die dritte Einfuhrstrecke, die dem Hub des Antriebsglieds 3 aus dem Gehäuse heraus entspricht, entspricht auch der erforderlichen Vorschubstrecke beim Vorschub des Antriebsglieds in distale Richtung zur Ausschüttung des Produkts. Die dritte Einfuhrstrecke entspricht somit einem Dosierhub für die Verabreichungsvorrichtung. Entsprechend dem erforderlichen Dosierhub, kann die Einfuhrstrecke daher bei der Konstruktion der Verabreichungsvorrichtung variiert werden.

In Figur 4B ist die Detailansicht analog der Figur 1B gezeigt, in der sich die Verabreichungsvorrichtung in einer eingeführten Position befindet. Beim Anheben des Antriebsglieds 3 aus dem Gehäuse 1 wird auch der Klemmsitz zwischen dem Sicherungselement 10 und den Vorsprung 11 überwunden.

Das Aufnahmeglied 2 ist nun vollständig in das Gehäuse 1 eingeschraubt, der Wirkstoff ist abgemischt, und die Produktkammer ist entlüftet. Die Verabreichungsvorrichtung wurde in einen aktivierten Zustand mit ausgefahrenem Antriebsknopf gebracht. Sie ist nun bereit, die gewünschte Dosis des Wirkstoffes zu verabreichen.

Hierfür wird, wie in Figur 5A dargestellt, das Aufnahmeglied 2 in das Gehäuse 1 in distale Richtung eingeschoben, wobei das Aufnahmeglied 2 gegenüber dem Gehäuse 1 in Ruhe bleibt. Diese Vorschubbewegung wird daher auf die Stopfen 4b und 4d übertragen, so dass das fluide Produkt aus der Produktkammer 4a ausgeschüttet wird. Das Antriebsglied 3 wird solange in dem Gehäuse vorgeschoben, bis der Antriebsknopf 8 gegen den Gehäusevorsprung 9 anschlägt. Dabei gleitet das Aktivierungselement 7, das durch den Vorsprung 11 nach innen ausgelenkt wurde, an der Innenseite des Aufnahmeglieds 2 in das Aufnahmeglied 2 hinein. Das Halteglied 6 gleitet ebenfalls entlang der Innenfläche des Aufnahmeglieds 2. Dabei wird der Absatz 6a entlang der Umfangsfläche des Aufnahmeglieds 2 geführt. Sobald der Antriebsknopf 8 gegen den Gehäusevorsprung 9 anschlägt, ist auch das proximale Ende des Halteglieds 6 an dem Gehäusevorsprung 9 vorbei bewegt worden. In der Innenumfangsfläche des Aufnahmeglieds 2 ist in der Höhe des Absatzes 6a eine Aussparung vorgesehen, in die der Absatz 6a ausweichen kann. Durch die Vorspannung des Halteelements 6 springt dieses in die Aussparung hinein und wieder in die Halteposition, in der das distale Ende des Halteelements 6 gegen den Gehäusevorsprung 9 anstösst. Der Antriebsknopf 8 ist wieder innerhalb des Gehäuses 1 versenkt.

Die Verabreichungsvorrichtung befindet sich nun in einer Endposition. Das Halteelement 6 ist dabei wieder in einer Halteposition, sodass das Antriebsglied 3 gegenüber dem Gehäuse 1 festgehalten wird. Die Verabreichungsvorrichtung ist daher gegen jegliche weitere Betätigung blockiert. In Figur 5B ist das Detail aus Figur 1B mit der Verabreichungsvorrichtung in einer Endposition gezeigt. Es ist ersichtlich, dass das Sicherungselement 10 wieder einen Klemmsitz mit dem Vorsprung 11 des Gehäuses 1 eingeht. Dadurch wird zusätzlich zu dem Haltegriff zwischen Halteelement 6 und Gehäusevorsprung 9 das Antriebsglied 3 gegen weitere Bewegungen oder ein Wackeln gesichert. Die Verabreichungsvorrichtung kann nun in diesem gesicherten Zustand entsorgt werden.

Es wird ausdrücklich darauf hingewiesen, dass es für den Zweck der vorliegenden Erfindung ausreicht, das Antriebsglied 3 durch das Halteelement 6 gegen ein proximales Bewegen relativ zum Gehäuse zu sichern. Das Sicherungselement 10 dient lediglich einer komfortableren Bedienung und einer zusätzlichen Führung für das Antriebsglied 3.

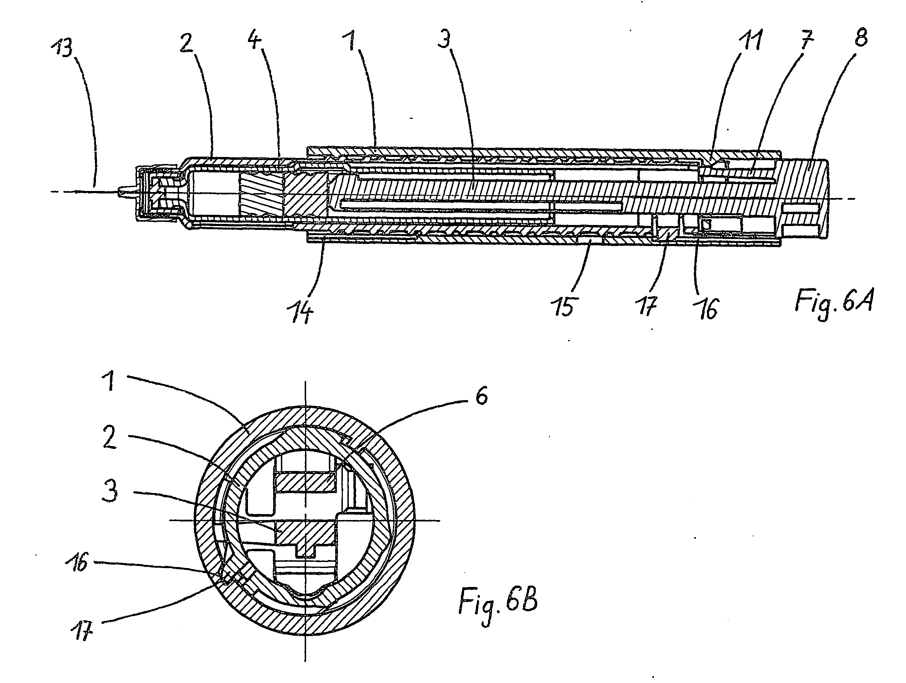

In Figur 6A ist ein Schnitt durch eine erfindungsgemässe Verabreichungsvorrichtung gezeigt, aus welcher die Rasteinrichtung, bzw. Anzeigenvorrichtung zur Verrastung und Anzeige bei den einzelnen Vorbereitungsschritten der Verabreichungsvorrichtung gezeigt ist. In Figur 6A ist die Verabreichungsvorrichtung bereits in einer Abmischposition. Die Anzeigen- oder Rasteinrichtung umfasst eine erste Rastrille 14, eine zweite Rastrille 15, die auch als Rastöffnung ausgebildet sein kann, und eine dritte Rastrille 16. Am Aufnahmeglied 2 ist ein in Umfangsrichtung ausgerichteter Rastarm 17 vorgesehen, der derart radial nach aussen vorgespannt ist, dass er beim Eindrehen des Aufnahmeglieds 2 in das Gehäuse 1 entlang der Innenumfangsfläche des Gehäuses gleitet. Beim Einsetzen des Aufnahmegliedes 2 in das Gehäuse 1 wird das Aufnahmeglied 2 soweit in das Gehäuse 1 eingedreht, bis der Rastarm 17 mit der ersten Rastrille 14 verrastet. In dieser Verrastung befindet sich die Verabreichungsvorrichtung in der Anfangsposition. Der Rastarm 17 ist derart ausgebildet, dass er auf Grund seiner Vorspannung ein Wiederherausdrehen, bzw. ein Zurückdrehen, des Aufnahmeglieds 2 aus dem Gehäuse heraus durch Anschlag an die erste Rastrille 14 blockiert. Aus dem Anfangszustand ist es daher nur möglich das Aufnahmeglied 2 weiter in proximale Richtung in das Gehäuse 1 einzudrehen. Grundsätzlich ist es möglich, die erste Rastrille derart über die Länge des Gehäuses anzuordnen, dass nach jeder Umdrehung des Aufnahmeglieds eine Verrastung eintritt. Mit der Verrastung zwischen dem Rastarm 17 und der Rastrille entsteht eine Anzeige, dass der Anfangszustand erreicht ist. Aus dem Anfangszustand wird das Aufnahmeglied weiter eingeführt bis es in der zweiten Rastöffnung 15 mit dem Rastarm 17 verrastet, wodurch die Beendigung des Abmischens des Zweikammerreservoirs angezeigt wird. Ist diese zweite Rastrille als Öffnung ausgebildet, ist es grundsätzlich möglich, dass der Rastarm 17 von aussen aus der Rastöffnung ins Innere eingedrückt wird, wodurch das Aufnahmeglied auch wieder aus dem Gehäuse 1 herausgedreht werden könnte. Diese Möglichkeit erscheint grundsätzlich sinnvoll, wenn nach dem Abmischen nicht unmittelbar eine Verabreichung aus der Verabreichungsvorrichtung erfolgen soll. Im Allgemeinen, wird diese zweite Rastrille jedoch nicht als Öffnung ausgebildet, so dass auch diese zweite Verrastung eine Rückdrehsicherung für das Aufnahmeglied aus dem Gehäuse darstellt. Aus dieser Abmischposition kann das Aufnahmeglied weiter in proximaler Richtung in das Gehäuse eingeschraubt werden, bis der Rastarm 17 in der dritten Rastrille verrastet ist, wodurch die Entlüftungsposition angezeigt wird. Die Verrastung zwischen dem Rastarm 17 und der dritten Rastrille 16 bildet gleichfalls eine Rückdrehsicherung, so dass das Aufnahmeglied in dieser Position gegen jegliche weitere Bewegung gegenüber dem Gehäuse blockiert ist, da die Aufnahmehülse 2 zugleich gegen den Gehäusevorsprung 9 in axialer Richtung anschlägt.

In Figur 6B ist ein Querschnitt durch die Verabreichungsvorrichtung gezeigt, in welcher der Rastarm 17 das Aufnahmeglied 2 in einer Rastposition mit einer Rastrille im Gehäuse 1 gezeigt ist. Aus Figur 6B ist ersichtlich, dass ein Verdrehen des Aufnahmegliedes 2 entgegen dem Uhrzeigersinn durch die Rastung verhindert wird, wohingegen ein Drehen des Aufnahmeglieds 2 innerhalb des Gehäuses 1 im Uhrzeigersinn ermöglicht wird, in dem der Rastarm 17 flexibel nach innen ausweicht und entlang der Schräge der Rille aus der Rille herausgleiten kann.

### Bezugszeichen:

- 1: Gehäuse
- 2: Aufnahmeglied
- 3: Antriebsglied
- 4: Produktreservoir
- 4a: Erste Kammer
- 4b: Erster Stopfen
- 4c: Zweite Kammer
- 4d: Zweiter Stopfen
- 5a: Führungsnut
- 5b: Führungsnocken
- 6: Halteelement
- 6a: Absatz
- 7: Aktivierungselement
- 8: Antriebsknopf
- 9: Gehäusevorsprung
- 10: Sicherungselement
- 11: Vorsprung
- 12: Bypass
- 13: Injektionsnadel
- 14: erste Rastrille
- 15: zweite Rastöffnung
- 16: dritte Rastrille
- 17: Rastarm

- 21: erste Anzeige
- 22: zweite Anzeige
- 23: Aussparung

## Patentansprüche

1. Vorrichtung zur Verabreichung eines fluiden Produkts, umfassend
a) ein Gehäuse (1)
b) ein Aufnahmeglied (2) für das fluide Produkt, bzw. für ein Reservoir (4) für das fluide Produkt, das relativ zum Gehäuse (1) entlang einer Längsachse des Gehäuses von einer Anfangsposition in eine eingeführte Position eingeführt werden kann,
c) ein Antriebsglied (3), das in dem Gehäuse (1) gelagert ist und relativ zum Aufnahmeglied (2) zur Ausschüttung des fluiden Produkts aus dem Aufnahmeglied (2) bzw. dem Reservoir (4) beweglich ist, wobei
d) das Antriebsglied (3) wenigstens ein Halteelement (6) besitzt, das in der Anfangsposition des Aufnahmeglieds (2) das Antriebsglied (3) relativ zum Gehäuse (1) hält, und durch Einführen des Aufnahmeglieds (2) das Haltelement (6) von der Halteposition in eine gelöste Position bewegt wird, in dem das Antriebsglied (3) relativ zum Gehäuse (1) beweglich ist, **dadurch gekennzeichnet, dass**
e) das Halteelement (6) in der Halteposition derart ausgebildet ist, dass das Antriebsglied (3) beim Einführen des Aufnahmeglieds (2) gegen den zweiten Stopfen (4d) stösst und diesen relativ zum Aufnahmeglied (2) beim Einführen verschiebt bis das Aufnahmeglied (2) in einer Abmischposition oder einer Entlüftungsposition ist.

2. Verabreichungsvorrichtung nach Anspruch 1, **dadurch gekennzeichnet, dass** das Halteelement (6) zum Halten des Antriebsglieds (3) mit dem Gehäuse (1) gekoppelt ist und zum Lösen des Antriebsglieds (3) vom Gehäuse entkoppelt ist.

3. Verabreichungsvorrichtung nach Anspruch 1 oder 2, **dadurch gekennzeichnet, dass** das Halteelement (6) zum Halten des Antriebsglieds gegen einen gehäusefesten axialen Anschlag (9) stösst.

4. Verabreichungsvorrichtung nach einem der vorhergehenden Ansprüche, **dadurch gekennzeichnet, dass** das Halteelement (6) als wenigstens ein radial vom Antriebsglied (3) abragender flexibler Fortsatz ausgebildet ist.

5. Verabreichungsvorrichtung nach dem vorherigen Anspruch, **dadurch gekennzeichnet, dass** der flexible Fortsatz aus der Halteposition gegen eine Spannkraft in die gelöste Position beweglich ist.

6. Verabreichungsvorrichtung nach Anspruch 4 oder 5, **dadurch gekennzeichnet, dass** der flexible Fortsatz in Richtung der Einführbewegung von dem Halteelement (6) abragt.

7. Verabreichungsvorrichtung nach einem der vorhergehenden Ansprüche, **dadurch gekennzeichnet, dass** beim Einführen des Aufnahmeglieds (2) das Aufnahmeglied (2) an dem Halteelement (6) angreift und in die gelöste Position bewegt.

8. Verabreichungsvomchtung nach einem der vorhergehenden Ansprüche, **dadurch gekennzeichnet, dass** das Reservoir (4) als Zweikammerreservoir ausgebildet ist, umfassend eine erste Kammer (4a) für einen Wirkstoff, die durch einen ersten Stopfen (4b) begrenzt ist, und eine zweite Kammer (4c) für ein Lösungsmittel, die durch den ersten Stopfen (4b) und einen zweiten Stopfen (4d) begrenzt ist, wobei die Kammern durch Verschiebung der Stopfen via einem Bypass verbindbar sind zum Abmischen des Wirkstoffs und des Lösungsmittels.

9. Verabreichungsvorrichtung nach einem der vorhergehenden Ansprüche, **dadurch gekennzeichnet, dass** das Antriebsglied (3) ein Aktivierungselement (7) aufweist, an dem das Aufnahmeglied (2) bei der Einführbewegung angreift und das Antriebsglied (3) relativ zum Gehäuse (1) in eine aktivierte Position bewegt.

10. Verabreichungsvorrichtung nach dem vorherigen Anspruch, **dadurch gekennzeichnet, dass** das Aktivierungselement (7) an einen Axialanschlag am Aufnahmeglied (2) bei der Einführbewegung anstösst.

11. Verabreichungsvorrichtung nach Anspruch 9 oder 10, **dadurch gekennzeichnet, dass** das Antriebsglied (3) in der aktivierten Position proximal aus dem Gehäuse ragt.

12. Verabreichungsvorrichtung nach einem der Ansprüche 9-11, **dadurch gekennzeichnet, dass** das Aktivierungselement (7) in einer Endposition nach einer Verabreichung des fluiden Produkts innerhalb des Aufnahmeglieds liegt.

13. Verabreichungsvorrichtung nach einem der vorhergehenden Ansprüche, **dadurch gekennzeichnet, dass** das Halteelement (6) in der Endposition gegen den gehäusefesten Anschlag (9) stösst.

14. Verabreichungsvorrichtung nach einem der vorhergehenden Ansprüche, **dadurch gekennzeichnet, dass** das Halteelement (6) in der Endposition im Wesentlichen innerhalb des Aufnahmegliedes (2) liegt.

15. Verabreichungsvorrichtung nach einem der vorhergehenden Ansprüche, **dadurch gekennzeichnet, dass** das Haltelement (6) einen Absatz (6a) umfasst, der eine Bewegung des Antriebsglieds (3) in proximale Richtung relativ zum Gehäuse durch Anschlag an einer gehäusefesten Kante (9) begrenzt.

16. Verabreichungsvorrichtung nach dem vorhergehenden Anspruch, **dadurch gekennzeichnet, dass** das Aufnahmeglied (2) hülsenförmig ausgebildet ist und eine Aussparung aufweist, in die der Absatz (6a) des Halteelements (6) in der Endposition eingreift.

17. Verfahren zur Vorbereitung einer Verabreichungsvorrichtung für ein fluides Produkt mit einer Steuereinrichtung, das folgende Schritte umfasst:
a. Lösen der Halteposition durch Einführen des Aufnahmeglieds (2) in proximaler Richtung in das Gehäuse (1) und
b. Aktivieren des Antriebglieds (3) durch Einführen des Aufnahmeglieds (2) in Einführrichtung in eine eingeführte Position,
**gekennzeichnet durch**
c. Einführen eines Aufnahmegliedes (2), bzw. Reservoirs (4), in Einführrichtung in ein Gehäuse (1) von einer Ausgangsposition bis zu einer Entlüftungsposition, wobei ein Antriebsglied (3) relativ zum Gehäuse (1) in einer Halteposition gehalten wird und einen Stopfen (4b; 4d) in dem Aufnahmeglied (2) zur Entlüftung vorschiebt.

18. Verfahren nach dem vorhergehenden Anspruch, **gekennzeichnet durch**
- Einführen des Aufnahmeglieds (2) von der Ausgangsposition in eine Abmischposition, wobei während dem Einführen ein Abmischen innerhalb eines Zweikammerreservoirs (4) erfolgt **durch** Vorschub eines Stopfens (4b; 4d) **durch** das Antriebsglied (3) und
- weiteres Einführen des Aufnahmeglieds (2) von der Abmischstellung in die Entlüftungsstellung.

19. Verfahren zur Vorbereitung einer Verabreichungsvorrichtung für ein fluides Produkt mit einer Steuereinrichtung, das folgende Schritte umfasst:
a. Lösen der Halteposition durch Einführen des Aufnahmeglieds (2) in proximaler Richtung in das Gehäuse (1) und
b. Aktivieren des Antriebglieds (3) durch Einführen des Aufnahmeglieds (2) in Einführrichtung in eine eingeführte Position,
**gekennzeichnet durch**
c. Einführen eines Aufnahmegliedes (2), bzw. Reservoirs (4), in Einführrichtung in ein Gehäuse (1) von einer Ausgangsposition bis zu einer Abmischposition, wobei ein Antriebsglied (3) relativ zum Gehäuse (1) in einer Halteposition gehalten wird und einen Stopfen (4b; 4d) in dem Aufnahmeglied (2) zum Abmischen vorschiebt.

## Claims

1. A device for administering a fluid product comprising
a) a housing (1),
b) a receiving member (2) for the fluid product or for a reservoir (4) for the fluid product, which can be introduced relative to the housing (1) along a longitudinal axis of the housing from an initial position into an introduced position,
c) a drive member (3) which is mounted in the housing (1) and is movable relative to the receiving member (2) for expelling the fluid product from the receiving member (2) or the reservoir (4), wherein
d) the drive member (3) has at least one holding element (6) which holds the drive member (3) relative to the housing (1) in the initial position of the receiving member (2) and the holding element (6) is movable by the introduction of the receiving member (2) from the holding position into a released position in which the drive member (3) is movable relative to the housing (1),
**characterised in that**
e) the holding element (6) in the holding position is so adapted that upon introduction of the receiving member (2) the drive member (3) butts against the second stopper (4d) and pushes it relative to the receiving member (2) in the introduction operation until the receiving member (2) is in a mixing position or a venting position.

2. An administration device according to claim 1 **characterised in that** the holding element (6) is coupled to the housing (1) for holding the drive member (3) and is uncoupled from the housing for releasing the drive member (3).

3. An administration device according to claim 1 or claim 2 **characterised in that** the holding element (6) for holding the drive member butts against an axial abutment (9) fixed with respect to the housing.

4. An administration device according to one of the preceding claims **characterised in that** the holding element (6) is in the form of at least one flexible extension projecting radially from the drive member (3).

5. An administration device according to the preceding claim **characterised in that** the flexible extension is movable out of the holding position into the released position against a stressing force.

6. An administration device according to claim 4 or claim 5 **characterised in that** the flexible extension projects in the direction of the introduction movement from the holding element (6).

7. An administration device according to one of the preceding claims **characterised in that** upon introduction of the receiving member (2) the receiving member (2) engages the holding element (6) and moves it into the released position.

8. An administration device according to one of the preceding claims **characterised in that** the reservoir (4) is in the form of a two-chamber reservoir including a first chamber (4a) for an active substance which is delimited by a first stopper (4b) and a second chamber (4c) for a solvent which is delimited by the first stopper (4b) and a second stopper (4d), wherein the chambers can be connected by displacement of the stoppers by way of a bypass for mixing of the active substance and the solvent.

9. An administration device according to one of the preceding claims **characterised in that** the drive member (3) has an activation element (7) which is engaged by the receiving member (2) in the introduction movement and moves the drive member (3) relative to the housing (1) into an activated position.

10. An administration device according to the preceding claim **characterised in that** the activation element (7) butts against an axial abutment on the receiving member (2) in the introduction movement.

11. An administration device according to claim 9 or claim 10 **characterised in that** the drive member (3) in the activated position projects proximally out of the housing.

12. An administration device according to one of claims 9 to 11 **characterised in that** the activation element (7) is disposed in an end position within the receiving member after administration of the fluid product.

13. An administration device according to one of the preceding claims **characterised in that** the holding element (6) butts in the end position against the abutment (9) which is fixed with respect to the housing.

14. An administration device according to one of the preceding claims **characterised in that** the holding element (6) is disposed in the end position substantially within the receiving member (2).

15. An administration device according to one of the preceding claims **characterised in that** the holding element (6) has a shoulder (6a) which limits a movement of the drive member (3) in the proximal direction relative to the housing by abutting against an edge (9) which is fixed with respect to the housing.

16. An administration device according to the preceding claim **characterised in that** the receiving member (2) is of a sleeve-shaped configuration and has an opening into which the shoulder (6a) of the holding element (6) engages in the end position.

17. A method of preparing an administration device for a fluid product having a control device which includes the following steps:
a. releasing the holding position by introduction of the receiving member (2) into the housing (1) in the proximal direction, and
b. activating the drive member (3) by introduction of the receiving member (2) in the introduction direction into an introduced position,
**characterised by**
c. introduction of a receiving member (2) or reservoir (4) in the introduction direction into a housing (1) from an initial position to a venting position, wherein a drive member (3) is held relative to the housing (1) in a holding position and advances a stopper (4b; 4d) in the receiving member (2) for venting purposes.

18. A method according to the preceding claim **characterised by**
- introduction of the receiving member (2) from the initial position into a mixing position, wherein during the introduction movement mixing is effected within a two-chamber reservoir (4) by forward movement of a stopper (4b; 4d) by the drive member (3), and
- further introduction of the receiving member (2) from the mixing position into the venting position.

19. A method of preparing an administration device for a fluid product having a control device which includes the following steps:
a. releasing the holding position by introduction of the receiving member (2) into the housing (1) in the proximal direction, and
b. activating the drive member (3) by introduction of the receiving member (2) in the introduction direction into an introduced position,
**characterised by**
c. introduction of a receiving member (2) or reservoir (4) in the introduction direction into a housing (1) from an initial position to a mixing position, wherein a drive member (3) is held relative to the housing (1) in a holding position and advances a stopper (4b; 4d) in the receiving member (2) for mixing purposes.

## Revendications

1. Dispositif d'administration d'un produit fluide, comprenant
a) un boîtier (1),
b) un élément de réception (2) pour le produit fluide, respectivement pour un réservoir (4) pour le produit fluide, qui peut être introduit par rapport au boîtier (1) le long d'un axe longitudinal du boîtier d'une position initiale à une position introduite,
c) un élément d'entraînement (3) logé dans le boîtier (1) et mobile par rapport à l'élément de réception (2) pour le déversement du produit fluide depuis l'élément de réception (2) respectivement du réservoir (4), dans lequel
d) l'élément d'entraînement (3) possède au moins un élément de retenue (6) qui maintient l'élément d'entraînement (3) par rapport au boîtier (1) dans la position initiale de l'élément de réception (2), et par l'introduction de l'élément de réception (2), l'élément de retenue (6) est déplacé de la position de retenue à une position détachée, dans laquelle l'élément d'entraînement (3) est déplaçable par rapport au boîtier (1), **caractérisé en ce que**
e) l'élément de retenue (6) est réalisé dans la position de retenue de telle manière que l'élément d'entraînement (3) bute lors de l'introduction de l'élément de réception (2) contre le deuxième bouchon (4d) et le déplace par rapport à l'élément de réception (2) lors de l'introduction jusqu'à ce que l'élément de réception (2) occupe une position de mélange ou une position d'aération.

2. Dispositif d'administration selon la revendication 1, **caractérisé en ce que** l'élément de retenue (6) est couplé au boîtier (1) pour la retenue de l'élément d'entraînement (3) et est découplé de celui-ci pour le détachement de l'élément d'entraînement (3).

3. Dispositif d'administration selon la revendication 1 ou 2, **caractérisé en ce que** l'élément de retenue (6) bute contre une butée axiale (9) fixée au boîtier pour la retenue de l'élément d'entraînement.

4. Dispositif d'administration selon l'une quelconque des revendications précédentes, **caractérisé en ce que** l'élément de retenue (6) est réalisé comme au moins un prolongement flexible dépassant radialement de l'élément d'entraînement (3).

5. Dispositif d'administration selon la revendication précédente, **caractérisé en ce que** le prolongement flexible est déplacé de la position de retenue à la position détachée contre une force de serrage.

6. Dispositif d'administration selon la revendication 4 ou 5, **caractérisé en ce que** le prolongement flexible dépasse de l'élément de retenue (6) dans le sens du mouvement d'introduction.

7. Dispositif d'administration selon l'une quelconque des revendications précédentes, **caractérisé en ce que** lors de l'introduction de l'élément de réception (2), l'élément de réception (2) agit sur l'élément de retenue (6) et le déplace dans la position détachée.

8. Dispositif d'administration selon l'une quelconque des revendications précédentes, **caractérisé en ce que** le réservoir (4) est réalisé comme un réservoir à deux chambres, comprenant une première chambre (4a) pour une substance active qui est délimitée par un premier bouchon (4b), et une deuxième chambre (4c) pour un solvant qui est délimitée par le premier bouchon (4b) et un deuxième bouchon (4d), les chambres pouvant être reliées par déplacement des bouchons via une dérivation pour le mélange de la substance active et du solvant.

9. Dispositif d'administration selon l'une quelconque des revendications précédentes, **caractérisé en ce que** l'élément d'entraînement (3) présente un élément d'activation (7), sur lequel agit l'élément de réception (2) lors du mouvement d'introduction et déplace l'élément d'entraînement (3) par rapport au boîtier (1) dans une position activée.

10. Dispositif d'administration selon la revendication précédente, **caractérisé en ce que** l'élément d'activation (7) bute contre une butée axiale sur l'élément de réception (2) lors du mouvement d'introduction.

11. Dispositif d'administration selon la revendication 9 ou 10, **caractérisé en ce que** l'élément d'entraînement (3) dépasse de manière proximale du boîtier dans la position activée.

12. Dispositif d'administration selon l'une quelconque des revendications 9 à 11, **caractérisé en ce que** l'élément d'activation (7) est dans une position finale après l'administration du produit fluide dans l'élément de réception.

13. Dispositif d'administration selon l'une quelconque des revendications précédentes, **caractérisé en ce que** l'élément de retenue (6) en position finale bute contre la butée fixée au boîtier (9).

14. Dispositif d'administration selon l'une quelconque des revendications précédentes, **caractérisé en ce que** l'élément de retenue (6) en position finale se situe sensiblement dans l'élément de réception (2).

15. Dispositif d'administration selon l'une quelconque des revendications précédentes, **caractérisé en ce que** l'élément de retenue (6) comporte un épaulement (6a) qui limite un déplacement de l'élément d'entraînement (3) dans le sens proximal par rapport au boîtier en butant contre une arête fixée au boîtier (9).

16. Dispositif d'administration selon la revendication précédente, **caractérisé en ce que** l'élément de réception (2) est réalisée en forme de douille et présente un évidement, dans lequel l'épaulement (6a) de l'élément de retenue (6) s'engage en position finale.

17. Procédé de préparation d'un dispositif d'administration pour un produit fluide avec un dispositif de commande, qui comporte les étapes suivantes :
a. le détachement de la position de retenue par l'introduction de l'élément de réception (2) dans le sens proximal dans le boîtier (1) et
b. l'activation de l'élément d'entraînement (3) par l'introduction de l'élément de réception (2) dans le sens d'introduction dans une position introduite,
**caractérisé par**
c. l'introduction d'un élément de réception (2), respectivement du réservoir (4), dans le sens d'introduction dans un boîtier (1) d'une position de sortie à une position d'aération, un élément d'entraînement (3) étant maintenu par rapport au boîtier (1) dans une position de retenue et poussant un bouchon (4b ; 4d) dans l'élément de réception (2) pour l'aération.

18. Procédé selon la revendication précédente, **caractérisé par**
- l'introduction de l'élément de réception (2) de la position de sortie à une position de mélange, pendant l'introduction, un mélange étant effectué dans un réservoir à deux chambres par avancement d'un bouchon (4b ; 4d) par l'élément d'entraînement (3) et
- la poursuite de l'introduction de l'élément de réception (2) de la position de mélange à la position d'aération.

19. Procédé de préparation d'un dispositif d'administration pour un produit fluide avec un dispositif de commande, qui comporte les étapes suivantes :
a. le détachement de la position de retenue par l'introduction de l'élément de réception (2) dans le sens proximal dans le boîtier (1) et
b. l'activation de l'élément d'entraînement (3) par l'introduction de l'élément de réception (2) dans le sens d'introduction dans une position introduite, **caractérisé par**
c. l'introduction d'un élément de réception (2), respectivement du réservoir (4), dans le sens d'introduction dans un boîtier (1) d'une position de sortie à une position de mélange, un élément d'entraînement (3) étant maintenu par rapport au boîtier (1) dans une position de retenue et poussant un bouchon (4b ; 4d) dans l'élément de réception (2) pour le mélange.
